# EUROPEAN PATENT SPECIFICATION

(11) **EP 1 485 499 B1**
(45) Date of publication and mention of the grant of the patent: **29.04.2009**
(21) Application number: 02717018.2
(22) Date of filing: 25.03.2002
(51) Int. Cl.: C12Q 1/68

(54) **NOVEL PRIMERS FOR SCREENING SCHIZOPHRENIA AND A METHOD THEREOF**
VERFAHREN UND NEUE PRIMER ZUR UNTERSUCHUNG VON SCHIZOPHRENIE
NOUVELLES AMORCES DE CRIBLAGE DE LA SCHIZOPHRENIE ET METHODE ASSOCIEE

(43) Date of publication of application: 15.12.2004
(73) Proprietor: Council of Scientific and Industrial Research, New Delhi 110 001 (IN)
(72) Inventor: BRAHMACHARI, Samir, Kumar, 110 007 Delhi (IN); RANJANA, Verma, 110 007 Delhi (IN); CHITRA, Chauhan, 110 007 Delhi (IN); Q., Salim, 110 007 Delhi (IN); S., Jain, 110 007 Delhi (IN)
(74) Representative: Atkinson, Peter Birch
(86) International application number: PCT/IB2002/001213
(87) International publication number: WO 2003/080867

(56) References cited:
- WO-A-99/64626
- DATABASE GENBANK [Online] 12 December 1999 (1999-12-12) COVILLE: "Human DNA sequence from clone RP-333H23" Database accession no. AL022326 XP002225578
- LIU ET AL.: "Genetic variation at the 22q11 PRODH2/DGCR6 locus presents an unusual pattern and increases susceptibility to schizophrenia" PROCEEDINGS OF THE NATIONAL ACADEMY OF SCIENCE, vol. 99, no. 6, 16 March 2002 (2002-03-16), pages 3717-3722, XP002225577

## Description

### Technical Field

The present invention relates to novel primers useful for identifying and screening non-sense mutation with codon TGG coding for amino acid tryptophan substituted with TAG a non-sense codon at nucleotide No. 825 in exon 2 of synaptogyrin 1 gene of chromosome 22q11-13, thereby detecting pre-disposition to schizophrenia in a subset of patients and a method thereof.

### Background art

Schizophrenia is a common and devastating illness afflicting at least 1% of the population worldwide. The characteristic symptoms involve disturbances in perception and inference (hallucinations and delusions), abnormalities in language, behaviour and motor function (disorganized speech, bizarre behaviour and catatonia) and deficits in emotional capacity and drive (affective flattening, anhedonia and avolition).

Abnormal neurotransmission affecting the dopamine, serotonin, glutamine, gamma-aminobutyric acid, and cholecystokinin systems have been reported in schizophrenia (Wolf et al, 1993). These abnormalities may affect genes involved in neurotransmitter metabolism, such as Catechol-o-methyl transferase (COMT) (Lachman et al, 1996) and Tyrosine hydroxylase (Ref); neurotransmitter receptors such as dopamine receptors (Seeman et al, 1993), N-methyl-D-Aspartate (NMDA) receptor (Nudmamud et al, 2001) and serotonin receptors (Chiu et al, 2001); and neurotransmitter transporters such as dopamine transporter (Persico et al, 1997).

Neurodevelopmental abnormalities are also strongly implicated in schizophrenia, with reports of defects in neuronal cytoskeleton (Arnold et al, 1991), neuronal cytoarchitecture (Arnold et al, 1997) and migration, cellular polarity, and synaptic pruning (Arnold, 1999). Thus, in schizophrenia, at least two processes appear to be aberrant: neurotransmission and neuronal development primarily affecting the later stages of synapse formation.

Unfortunately, there is no objective laboratory test for schizophrenia, and the diagnosis is made by clinical interview. Since there is a continuing need for developing diagnostic methods and new therapies for such diseases, efforts have been devoted to the characterization and elucidation of the genes responsible for schizophrenia.

In an attempt to device a method for diagnosing schizophrenia, Meloni et al (USPT-6,210,879) used a microsatellite marker, HUMTH01 present in the first intron of tyrosine hydroxylase gene for finding association with schizophrenia. This marker consists of repeated tetrameric TCAT motifs. The most frequently encountered allele of the marker comprises 10 repeated motifs and a deletion of one base pair in the fifth repeated motif, which has the sequence CAT. However, while doing association analysis of this repeat with schizophrenia, they observed that the perfect repeat (without the deletion) was rare and only present in schizophrenic patients.

Although, not much is known about the cause of schizophrenia, the disease has a strong genetic component. Research into the genetics of schizophrenia reveals that this disease is heterogeneous and is a "complex genetic" disease, that is, several genes may be involved in the etiology of this disease.

Several genetic studies have reported significant linkage to several chromosomal regions, which include 1q21-22, 6p24-22, 7q, 8p22-21, 10p14-13, 13q32, 18p and 22q11-13 (Riley and McGuffin, 2000).

One of the most intensively studied regions amongst these includes several loci on chromosome 22. Initial genome wide scans for schizophrenia by different groups suggested possible linkage for markers on chromosome 22q although neither of the groups reported statistically significant results (Schwab et al, 1999; Coon et al, 1994; Pulver et al, 1994).

A combined transmission disequlibrium and linkage analysis of D22S278 in 574 families further strengthened the possibility of a susceptibility locus on chromosome 22q (Schizophrenia Collaborative Linkage group for chromosome 22). A further line of evidence implicating chromosome 22 in schizophrenia has come from the study of patients with a congenital malformation called Velo Cardio Facial Syndrome (VCFS). VCFS is known to be caused by deletions in the region of 22q11.2-q11.23 and patients suffering from this disorder show a high prevalence of psychiatric illnesses including both bipolar disorder and schizophrenia (Arnold, 2001). Taken together, these independent lines of evidence from cytogenetic studies and linkage analysis studies suggest that chromosome 22 might indeed harbour susceptibility loci for schizophrenia.

Apart from linkage studies, an alternative approach which has evoked a great deal of interest in the recent years has been the study of trinucleotide repeat expansions in bipolar disorder and schizophrenia (Vincent et al, 2000). Studies of anonymous CAG repeats using the Repeat Expansion Detection (RED) technique have demonstrated expanded repeats in schizophrenia and bipolar disorder with considerable overlap between patients and controls (O'Donovan et al, 1996; Morris et al, 1995).

A great deal of effort has focused on the identification of loci containing trinucleotide repeats as candidate genes for these diseases. The candidate gene approach, however, has been unable to demonstrate large expansions of trinucleotide repeats in the range of those seen in the diseases caused by triplet repeat expansions in patients suffering from schizophrenia and bipolar disorder (Vincent et al, 2000).

The failure to observe large expansions has led to suggestions that it might be worthwhile studying moderate trinucleotide repeat expansions in patients suffering from these diseases (Petronis et al, 1996).

Applicants have also proposed earlier that a difference in allele sizes or 'allele span' at such polymorphic trinucleotide repeat loci may also be implicated in bipolar disorder and schizophrenia (Saleem et al, 1998; Saleem et al, 2000).

As chromosome 22 has been repeatedly implicated in bipolar disorder and schizophrenia, susceptibility loci on this chromosome might contain expanded CAG repeats involved in the pathogenesis of these disorders. In order to identify such loci on chromosome 22, CAG repeats containing more than five repeats and mapping to schizophrenia susceptibility loci on chromosome 22 were identified and studied for the association with the disease. One of such CAG repeat markers, 22CH3, present on chromosome 22q11-13 was shown to be associated with schizophrenia in Indian population (Saleem et al, 2001). This locus is biallelic with 7 and 8 CAG repeats. The 8 repeat allele at this locus was significantly over represented in schizophrenic patients when compared to ethnically matched controls. The applicants further identified genes in the vicinity of this locus .Out of these, Synaptogyrinl was chosen as a candidate gene for schizophrenia since it plays an integral role in neurotransmitter release, thus, mutations in this gene could explain many of the defects observed in schizophrenia.

Two recent studies have reported mutations in two different genes in schizophrenic patients. One of them is a frameshift mutation in KCNN3 gene, located on chromosome 1q21-22, found in only one schizophrenic patient (Bowen et al, 2001). The mutation found to be associated with schizophrenia is a missense mutation in WKL 1 gene, present on 22q11-13, and is shown to be segregating with schizophrenia in only one large family (Meyer et al, 2001). Since schizophrenia is a multigenic disorder, so it is quite possible that this region may harbour some other genes also, which when mutated might lead to schizophrenia.

In a study involving microarray expression profiling of prefrontal cortex from matched pairs of patients with schizophrenia and control subjects, it was found that transcripts encoding proteins involved in the regulation of presynaptic function were decreased in all subjects with schizophrenia (Mimics, 2000).

Further Double knockout mice for Synaptogyrin 1 and synaptophysin genes have shown deficits in long-term and short-term synaptic plasticity (Roger et al, 1999). These studies only suggest that Synaptogyrin 1 is a potential candidate gene for conferring susceptibility to schizophrenia.

The applicants have consequently examined the role of Synaptogyrin 1 gene in conferring susceptibility to schizophrenia in Indian population. The human SYNGR1 gene reveals three (SYNGR1a, SYNGR1b, SYNGR1c) alternative transcript forms of 4.5, 1.3 and 0.9 kb, respectively. The most abundant SYNGR1 a transcript, the 4.5-kb form, which corresponds to RATSYNGR1, is highly expressed in neurons of the central nervous system and at much lower levels in other tissues, as determined by in situ hybridization histochemistry. The levels of SYNGR1b and SYNGR1c transcripts are low and limited to heart, skeletal muscle, ovary and fetal liver (Kedra et al, 1997).

ShaiKh et al. (Human Genetics, Vol. 97, No.6, June 1996, pages 714-719) discloses an association between a polymorphism in the dopamine D3 receptor gene and schizophrenia. liu et al. (Proceedings of the National Academy of Science, vol.99 No.6, March 2002 3717-3712) discloses genetic variation at the 22gII PRODH2/D9CR6 locus associate with schizophrenia

Schizophrenia is a multigenic disorder, so the identification of more candidate genes in addition to those already known is required for predicting the pre-disposition to schizophrenia rather than diagnosis only when the symptoms have already set in. This would help in devising life style changes and to have suitable environs for the subjects predisposed to the disease, which would be helpful in alleviating the severity of schizophrenia.

Present invention relates to a method of identification of mutation in Synaptogyrin 1 gene for detection of pre-disposition to schizophrenia. Utility of the invention lies in the detection of mutation in Synaptogyrin 1 gene, which has been envisaged to be responsible for susceptibility to schizophrenia. The invention also has the utility for developing therapeutics for treatment of schizophrenia, as well as the constructions of transgenic animals expressing the mutant gene. A CAG repeat marker (22CH3) on chromosome 22q11-13 has been found to be associated with schizophrenia in the Indian population. In the vicinity of this region lies the Synaptogyrin 1 gene, which plays an integral role in neurotransmission. More particularly, the present invention relates to the method of identifying mutations in Synaptogyrin 1 gene. The present invention also provides the specific primers, which can be used for detecting mutation in Synaptogyrin 1 gene. The present invention also relates to methods for diagnosing and detecting carriers of the mutation in Synaptogyrin 1 gene.

### Object of the present invention

The main object of the present invention is to develop probes and/or primers specific toward mutation site of schizophrenic patients.

Another main object of the present invention is to develop a method to identify genetic mutation in schizophrenia.

Yet another object of the present invention is to develop a screening method for patients suffering from schizophrenia.

Still another object of the present invention is to develop oligonucleotide primers specific for amplification of mutation comprising DNA stretch of schizophrenic patients.

Still another object of the present invention is to determine the location of the mutation in the human genome.

Still another object of the present invention is to determine inheritance pattern of schizophrenia in affected families.

Still another object of the present invention is to develop a diagnostic kit for identification and screening pre-disposition to schizophrenia.

### Summary of the present invention

The present invention relates to novel primers useful for identifying and screening non-sense mutation with codon TGG coding for amino acid tryptophan substituted with TAG a non-sense codon at nucleotide No. 825 in exon 2 of synaptogyrin 1 gene of chromosome 22q11-13, thereby detecting pre-disposition to schizophrenia in a subset of patients and a method thereof.

### Detailed description of the present invention:

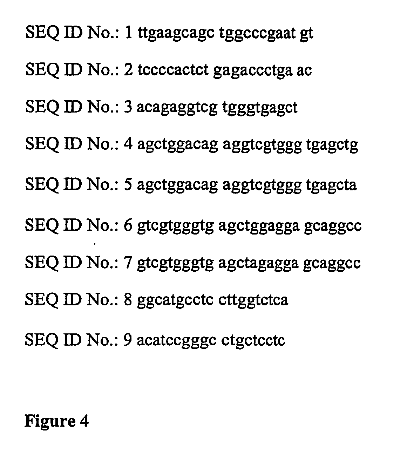

Accordingly, present invention relates to novel primers useful for identifying and screening non-sense mutation with codon TGG coding for amino acid tryptophan substituted with TAG a non-sense codon at nucleotide No. 825 in exon 2 of synaptogyrin 1 gene of chromosome 22q11-13, thereby detecting pre-disposition to schizophrenia in a subset of patients. (Please refer figure 4 for primers of SEQ ID Nos.1 to 9).

In one aspect of the present invention, there is provided primers of SEQ ID Nos. 1 and 2.

In another aspect of the present invention, there is provided the primer of SEQ ID No. 3.

In yet another aspect of the present invention, there is provided the primers and/or probes of SEQ ID Nos. 4-7.

Said primers are useful for amplifying exon 2 of the synaptogryin I gene.

SEQ ID NO.5 is designed to have a mutated base occupy a 3' position of the probe/primer.

SEQ ID NO.7 is designed to have a mutated base occupy a central position of the probe/primer.

According to a further aspect of the present invention there is provided an *in vitro* method of screening human beings for a pre-disposition to schizophrenia by identifying a polymorphism A/G at nucleotide No. 825 from 5'end in exon 2 and its allelic variants in synaptogyrin 1 gene of chromosome 22ql 1-13, for detecting non-sense mutation of codon TGG coding for the amino acid tryptophan to a TAG, a non-sense codon said method comprising:
(a) isolating DNA from blood leukocytes;
(b) amplifying isolated DNA by PCR using primers of SEQ ID No. 1 and 2
(c) sequencing the amplified DNA;
(d) comparing the sequenced DNA with that of normal synaptogyrin 1 gene;
(e) identifying the said mutation;
(f) designing oligonucleotide primer and/or probe of SEQ ID No. 3 of enclosed sequence listing with its 3'end extending upto penultimate position of said mutation;
(g) screening for said non-sense mutation using primer and/or probe of step (f); and
(h) screening of said allelic variation for said non-sense mutation using appropriate allele specific oligonucleotide probes and/or primers selected from a group comprising SEQ ID No. 4 to 7 of enclosed sequence listing.

The oligonucleotide primer and/or probe of SEQ ID NO. 3 of enclosed sequence listing is designed with its 3' end extending upto the penultimate position of said mutation.

Said method may be used to understand inheritance patterns in schizophrenic families.

The method allows early detection which helps manage disease before physiological manifestations set in.

The method may be used to screen affected individuals with said mutation in heterozygous state.

According to a further aspect of the present invention there is provided a diagnostic kit useful for screening human beings for detecting a pre-disposition to schizophrenia by identifying a polymorphism A/G at nucleotide No. 825 from 5'end in exon 2 and its allelic variants in synaptogyrin 1 gene of chromosome 22ql 1-13, for detecting non-sense mutation of codon TGG coding for the amino acid tryptophan to a TAG, a non-sense codon, wherein said kit comprises:
(a) primers of SEQ ID No. 1-2;
(b) probes and/or primers of SEQ ID No. 3-7, and
(c) other additives selected from restriction enzymes, reverse transcriptases, polymerases, ligases, linkers, nucleoside triphosphates as substrate, suitable buffers, labels, and/or other accessories.

The restriction enzymes may be *HpaII, HaeIII, BamHI, HpaI, EcoRI, HindIII and PvuII.*

The linkers may be selected from both sticky and blunt end types.

The nucleoside triphosphates may be selected from the group comprising adenine triphosphates, guanine triphosphates, cytosine triphosphates, and thymine triphosphates.

Direct sequencing of exons of this gene in the patient samples led to the discovery of nonsense mutation in second exon in one schizophrenic family. In the affected individuals of this family the codon TGG, which codes for amino acid tryptophan has been mutated to TAG, which is a nonsense codon. The mutation was present in the heterozygous state in the affected individuals, which could lead to the haploinsufficiency of the Synaptogyrin 1 gene product

Since Synaptogyrin 1 is involved in neurotransmission, a nonsense mutation in this gene might lead to susceptibility for schizophrenia. Further genotyping of 148 schizophrenic probands and 143 ethnically matched normal controls did not show this mutation. These results constitute the first demonstration of association of a nonsense mutation in a candidate gene, conferring susceptibility to schizophrenia.

A subset of schizophrenia patients may harbor this mutation or yet unknown mutation in this gene as it is often found that many different mutations localize to a given gene whose loss of function is responsible for the disease in a subset of patients.

The expression of exon 2 of Synaptogyrin 1 gene in brain may be confirmed by amplifying the region spanning the nonsense mutation in brain cDNA using exon 2 and exon 4 specific primers of SEQ IDs 8 and 9.
5' GGC ATG CCT CCT TGG TCT CA 3'(As listed in SEQ ID NO: 8)
5' CGT CCG TCC CTT CGT TCA 3' (As listed in SEQ ID NO: 9)

The primers suitable for amplifying exon 2 of Synaptogyrin 1 gene containing nonsense mutation are selected from the group consisting of SEQ ID NO: 1, SEQ ID NO: 2 and the compliments thereof.

The allele specific primers and probes useful for detection of nonsense mutation in Synaptogyrin 1 gene are selected from the group consisting of SEQ ID NO: 4, SEQ ID NO: 5, SEQ ID NO: 6, SEQ ID NO: 7 (wherein the mutated base occupies a central position of the probe).

The length of the oligonucleotide primers and probes are in the range of 5 to 100 bases.

Applicants carried out the PCR amplification of exon 2 of the human Synaptogyrin 1 gene using oligonucleotide primers. These primers were designed in accordance with the human Synaptogyrin 1 sequence submitted by Sanger Centre, Hinxton, Cambridgeshire, CB10 ISA, and UK. (08-DEC-1999) (GenBank accession number- AL022326).

Sequencing of the purified PCR product revealed nonsense mutation in exon 2 of human Synaptogyrin 1 gene in one schizophrenic family. It was apparent, therefore, that there is a hitherto unrecognized allele or subtype of the human Synaptogyrin 1 gene.

A sequence for the allelic variants of human Synaptogyrin 1 gene comprising nonsense mutation was compared with the human Synaptogyrin 1 gene sequence in the database (GenBank accession number-AL022326).

**Table 1**

| Site of change | Base change | Amino-acid alteration |
|---|---|---|
| 825 | G-A | Trp-nonsense codon |

The sites of changes were in accordance with the PCR Product Sequence obtained using primers (SEQ ID 1 and 2) flanking exon 2 of Synaptogyrin 1 gene The mutated site had either G or A. The substitution G→A changes amino acid tryptophan to nonsense codon which consequently leads the nucleotide sequence of the allelic variant of human Synaptogyrin 1 gene containing the nonsense mutation -

The mutated site is at nucleotide position 825 in said gene sequence.

Analysis of 20 densely affected schizophrenia families of South Indian origin as well as ethnically matched normal individuals revealed the presence of this non-sense mutation in one of the schizophrenic families. This family comprises of three schizophrenic patients and two normal individuals. Out of these, the nonsense mutation is present in heterozygous state in all three schizophrenic patients as well as in one of the normal individuals and it is absent in the other normal individual. The normal individual having the mutation may be asymptomatic at present as she is of younger age or this could be due to incomplete penetrance of the mutation. Further genotyping of 148 schizophrenic patients and 143 ethnically matched normal controls did not show this mutation.

To demonstrate the expression of exon2 (containing the nonsense mutation) in human brain, the applicants designed the exon 2 and exon 4 specific primers, isolated RNA from human brain, made cDNA and amplified region spanning the nonsense mutation in human brain cDNA. The exon 2 containing the nonsense mutation was found to be expressed in brain.

The kits according to the invention may contain one or more pairs of allele-specific oligonucleotides hybridizing to different forms of a polymorphism. In some kits, the allele-specific oligonucleotides are provided immobilized to a substrate, which can be used to detect mutation in Synaptogyrin 1 gene. Optional additional components of the kit include, for example, restriction enzymes, reverse-transcriptase or polymerase, the substrate nucleoside triphosphates, means used to label (for example, an avidin enzyme conjugate and enzyme substrate and chromogen if the label is biotin), and the appropriate buffers for reverse transcription, PCR, or hybridization reactions. Usually, the kit also contains instructions for carrying out the methods and also any other SNP detection reagent and method like Taqman.

### Brief descriptions of the accompanying drawings:

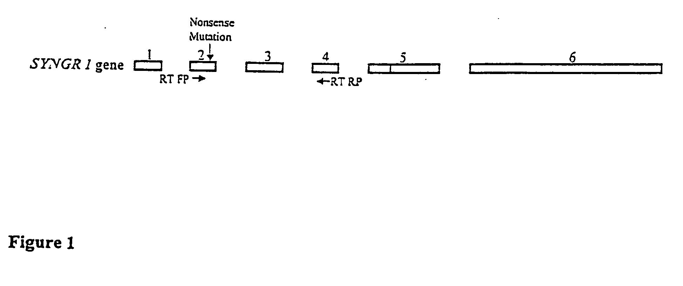

- **Fig 1**: shows schematic representation of the nonsense mutation in Synaptogyrin 1 gene. The top line depicts the position of the 6 exons of the Synaptogyrin 1 gene with the relative positions of nonsense mutation in the second exon, and the primers used for RT-PCR.

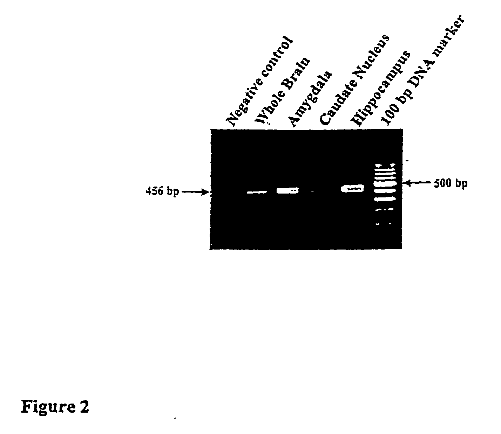

- **Fig 2**: shows the expression of exon 2 of Synaptogyrin 1 gene in different parts of brain by RT-PCR using exon 2 (SEQ ID 8) and exon 4 (SEQ ID 9) specific primers.

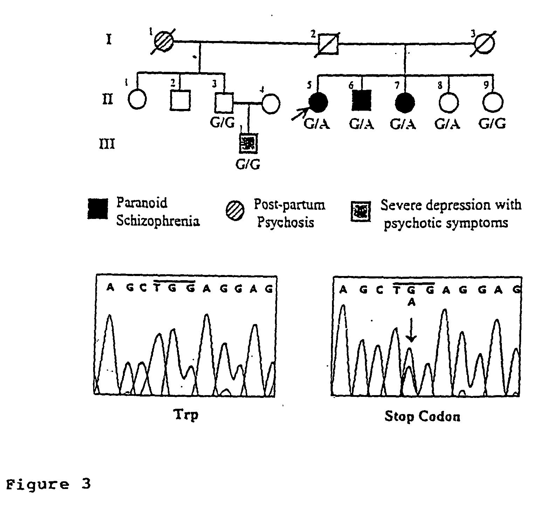

- **Fig 3**: shows the pedigree of the family in which the mutation has been found. The circles represent female members and squares represent male members. Filled circles and squares represent affected members of the family while open ones represent the normal family members. The horizontal line represents the marriage and the vertical line represent the next generation. The genotype of family members screened for the mutation is shown below the symbols. Below the pedigree is shown the electropherogram depicting the mutation in exon 2 of Synaptogyrin 1 gene.
- **Fig.4**: shows SEQ ID Nos. 1 to 9.

The following examples are given by way of illustration of the present invention and should construed to limit the scope of the present invention.

### Example 1

### Identification of nonsense mutation in Synaptogyrin 1 gene:

This example describes the identification of mutation in exon 2 of Synaptogyrin 1 gene by PCR and sequencing, using certain oligonucleotide primers according to the invention.

DNA was extracted from human peripheral blood leukocytes using a modification of the salting out procedure. The concentration of the DNA was determined by measuring the absorbance of the sample, at a wavelength of 260 nm. The DNA from schizophrenic probands was then amplified by polymerase chain reaction by using the oligonucleotide primer 1 and 2 (SEQ ID land 2). The samples were denatured at 94°C for 5 min followed by 35 cycles of denaturation 94°C, 30sec), annealing (67°C, 30sec), extension (72°C, 1 min) and a final extension of 7 min at 72°C in a Perkin Elmer Gene Amp PCR System 9600. This reaction produced a DNA fragment of 1057 bp. The PCR product was purified from band excised from agarose gel using a DNA ISOLATION KIT (Biological Industries, Israel) and both the strands of the PCR product were directly sequenced using dye terminator chemistry on an ABI Prism 377 automated DNA sequencer. The PCR product was shown to be identical to the exon 2 of the Synaptogyrin 1 gene sequence in the database (accession number-AL022326), except for the previously mentioned mutation in one schizophrenic patient.

### Example 2

### Screening mutation in the population:

This example describes a primer extension reaction used to screen single nucleotide variants. The DNA samples from 148 patients and 143 normal subjects were amplified by PCR and the PCR products were purified as described in example 1. The primer extension reaction was performed on the purified PCR products using oligonucleotide primer and SNaPshot ddNTP primer extension kit (PE Biosystems). The oligonucleotide primer was designed till the penultimate position of mutation and the primer is extended by one ddNTP, which is in accordance with the variant allele present. The reaction was performed for 25 cycles of denaturation (96°C, 10 sec), annealing (50°C, 5 sec) and extension (60°C, 30 sec) in a Perkin Elmer GeneAmp PCR System 9600. The primer extension products were treated with calf intestine alkaline phosphatase (New England Biolabs) for removing unincorporated dideoxynucleotides. The products were run on an ABI Prism 377 automated DNA sequencer. Depending on the colour of the fluoroscently labeled dideoxynucleotide incorporated, the wild-type and mutant alleles of the Synaptogyrin gene were detected.

### Example 3

### Nucleotide sequenced of allelic variants of Synaptogyrin 1 gene:

The nucleotide sequence of the allelic variant of Synaptogyrin 1 gene derived using the method as described in example 1

### Example 4

### The nonsense mutation is segregated along with disease in schizophrenic family:

The nonsense mutation was found in the affected members of one schizophrenic family. This family comprises of three schizophrenic patients and two normal individuals. Out of these, the nonsense mutation is present in heterozygous state in all the three schizophrenic patients as well as in one normal individual and it is absent in other normal individual. The normal individual having the mutation may be asymptomatic at present as she is of younger age or this could be due to incomplete penetrance of the mutation.

### Example 5

### Studying the expression of exon 2 of Synaptogyrin 1 gene in human brain:

To demonstrate the expression of exon2 (containing nonsense mutation) in human brain, the exon 2 and exon 4 specific primers were designed. RNA was isolated from human brain using EZ RNA Isolation kit (Biological Industries). cDNA was synthesized using random primers and oligo dT primers (1st strand cDNA synthesis kit for RT-PCR, Boehringer Mannheim). The region spanning the nonsense mutation in human brain cDNA was amplified using the exon 2 and exon4 specific primers. The exon 2 containing the nonsense mutation was found to be expressed in brain.

### References:

1. Arnold PD, Siegel-Bartelt J, Cytrynbaum C, Teshima I, Schachar R. (2001). Velo-cardio-facial syndrome: Implications of microdeletion 22q11 for schizophrenia and mood disorders. Am J Med Genet; 105(4): 354-62
2. Arnold SE, Lee VM, Gur RE, Trojanowski JQ. (1991). Abnormal expression of two microtubule-associated proteins (MAP2 and MAP5) in specific subfields of the hippocampal formation in schizophrenia. Proc Natl Acad Sci U S A; 88(23): 10850- 4
3. Arnold SE, Ruscheinsky DD, Han LY. (1997). Further evidence of abnormal cytoarchitecture of the entorhinal cortex in schizophrenia using spatial point pattern analyses. Biol Psychiatry; 42(8): 639-47
4. Arnold SE. (1999). Neurodevelopmental abnormalities in schizophrenia: insights from neuropathology. Dev Psychopathol; 11(3): 439-56
5. Bowen T, Williams N, Norton N, Spurlock G, Wittekindt OH, Morris-Rosendahl DJ, Williams H, Brzustowicz L, Hoogendoom B, Zammit S, Jones G, Sanders RD, Jones LA, McCarthy G, Jones S, Bassett A, Cardno AG, Owen MJ, O'Donovan MC. (2001). Mutation screening of the KCNN3 gene reveals a rare frameshift mutation. Mol Psychiatry 6(3): 259-60.
6. Chiu HJ, Wang YC, Liou JH, Chao CH, Lee H, Tsai KY, Liu WC. (2001). Serotonin 6 receptor polymorphism in schizophrenia: frequency, age at onset and cognitive function. Neuropsychobiology; 43(3): 113-6
7. Coon H, Jensen S, Holik J, Hoff M, Myles-Worsley M, Reimherr F, Wender P, Waldo M, Freedman R, Leppert M, et al. (1994). Genomic scan for genes predisposing to schizophrenia. Am J Med Genet Mar; 54(1): 59-71
8. Janz R, Sudhof TC, Hammer RE, Unni V, Siegelbaum SA, Bolshakov.VY. (1999). Essential roles in synaptic plasticity for Synaptogyrin 1 and Synaptophysin 1. Neuron; 24: 687-700
9. Kedra D, Pan H-Q, Seroussi E, Fransson I, Guilbaud C, Collins JE, Dunham I, Blennow E, Roe BA, Piehl F, Dumanski JP. (1998). Characterization of human Synaptogyrin gene family. Hum Genet; 103:131-141
10. Lachman HM, Papolos DF, Saito T, Yu YM, Szumlanski CL, Weinshilboum RM. (1996). Human catechol-O-methyltransferase pharmacogenetics: description of a functional polymorphism and its potential application to neuropsychiatric disorders. Pharmacogenetics; 6(3): 243-50
11. Meloni et al, 2001. Method for diagnosing schizophrenia. US Patent 6,210,879.
12. Meyer J, Huberth A, Ortega G, Syagailo YV, Jatzke S, Mossner R, Strom TM, Teuber IU, Stober G, Schmitt A, Lesch KP. (2001). A missense mutation in a novel gene encoding a putative cation channel is associated with catatonic schizophrenia in a large pedigree. Mol Psychiatry; 6: 302-306
13. Mimics K, Middleton FA, Marquez A, Lewis DA, Levitt P. (2000). Molecular characterization of schizophrenia viewed by microarray analysis of gene expression in prefrontal cortex. Neuron; 28: 53-67
14. Morris AG, Gaitonde E, McKenna PJ, Mollon JD, Hunt DM. (1995). CAG repeat expansions and schizophrenia: association with disease in females with early age-at-onset. Hum Mol Genet; 4(10): 1957-61
15. Nudmamud S, Reynolds GP. (2001). Increased density of glutamate/N-methyl-D-aspartate receptors in superior temporal cortex in schizophrenia. Neurosci Lett; 304(1-2): 9-12
16. O'Donovan MC, Guy C, Craddock N, Bowen T, McKeon P, Macedo A, Maier W, Wildenauer D, Aschauer HN, Sorbi S, Feldman E, Mynett-Johnson L, Claffey E, Nacmias B, Valente J, Dourado A, Grassi E, Lenzinger E, Heiden AM, Moorhead S Harrison D, Williams J, McGuffin P, Owen MJ. (1996). Confirmation of association between expanded CAG/CTG repeats and both schizophrenia and bipolar disorder. Psychol Med; 26(6): 1145-53
17. Persico AM, Macciardi F. (1997). Genotypic association between dopamine transporter gene polymorphisms and schizophrenia. Am J Med Genet; 74(1): 53-7.
18. Petronis A, Bassett AS, Honer WG, Vincent JB, Tatuch Y, Sasaki T, Ying DJ, Klempan TA, Kennedy JL. (1996). Search for unstable DNA in schizophrenia families with evidence for genetic anticipation. Am J Hum Genet; 59(4): 905-11
19. Pulver AE, Karayiorgou M, Wolyniec PS, Lasseter VK, Kasch L, Nestadt G, Antonarakis S, Housman D, Kazazian HH, Meyers D, et al. (1994). Sequential strategy to identify a susceptibility gene for schizophrenia: report of potential linkage on chromosome 22q12-q13.1: Part 1. Am J Med Genet; 54(1): 36-43
20. Riley BP, McGuffin P. (2000). Linkage and associated studies of schizophrenia. Am J Med Genet; 97(1): 23-44
21. Saleem Q, Vijayakumar M, Mutsuddi M, Chowdhary N, Jain S, Brahmachari SK. (1998). Variation at the MJD locus in the major psychoses. Am J Med Genet; 81(5): 440-2.
22. Saleem Q, Sreevidya VS, Sudhir J, Savithri JV, Gowda Y, B-Rao C, Benegal V, Majumder PP, Anand A, Brahmachari SK, Jain S. (2000). Association analysis of CAG repeats at the KCNN3 locus in Indian patients with bipolar disorder and schizophrenia. Am J Med Genet; 96(6): 744-8
23. Saleem Q, Dash D, Gandhi C, Kishore A, Benegal V, Sherrin T, Mukherjee O, Jain S and Brahmachari SK. (2001). Association of CAG repeat loci on chromosome 22 with schizophrenia and bipolar disorder. Mol Psychiatry (In Press)
24. Schizophrenia Colloborative Linkage Group For Chromosome 22. (1998). A transmission disequilibrium and linkage analysis of D22S278 marker alleles in 574 families: further support for a susceptibility locus for schizophrenia at 22q 12. Schizophrenia Research 32: 115-1
25. Schwab SG, Wildenauer DB. (1999). Chromosome 22 workshop report. Am J Med Genet; 88(3): 276-8
26. Seeman P, Guan HC, Van Tol HH. (1993). Dopamine D4 receptors elevated in schizophrenia. Nature; 365(6445): 441-5
27. Vincent JB, Paterson AD, Strong E, Petronis A, Kennedy JL. (2000). The unstable trinucleotide repeat story of major psychosis. Am J Med Genet; 97(1): 77-97
28. Wolf, SS, Hyde, TM and Weinberger, DR. (1993). Neurobiology of schizophrenia. Curr. Opinion Neurol. Neurosurg; 6: 86-92.

## Claims

1. The primers of SEQ ID Nos. 1 and 2.

2. The primer of SEQ ID No. 3.

3. The primers and/or probes of SEQ ID Nos. 4-7.

4. A use of the Primers as claimed in claim 1 for amplifying exon 2 of synaptogyrin 1 gene.

5. An *in vitro* method of screening human beings for a pre-disposition to schizophrenia, by identifying a polymorphism A/G at nucleotide No. 825 from 5'end in exon 2 and its allelic variants in synaptogyrin 1 gene of chromosome 22q1 1-13, for detecting non-sense mutation of codon TGG coding for the amino acid tryptophan to a TAG, a non-sense codon said method comprising:
(a) isolating DNA from blood leukocytes;
(b) amplifying isolated DNA by PCR using primers of SEQ ID No. 1 and 2
(c) sequencing the amplified DNA;
(d) comparing the sequenced DNA with that of normal synaptogyrin 1 gene;
(e) identifying the said mutation;
(f) designing oligonucleotide primer and/or probe of SEQ ID No. 3 of enclosed sequence listing with its 3'end extending upto penultimate position of said mutation;
(g) screening for said non-sense mutation using primer and/or probe of step (f); and (h) screening of said allelic variation for said non-sense mutation using appropriate allcle specific oligonucleotide probes and/or primers selected from a group comprising SEQ ID No. 4 to 7 of enclosed sequence listing.

6. The method as claimed in claim 5, for studying inheritance patterns in schizophrenic families.

7. The method as claimed in claim 5, wherein the method allows early detection to allow management of the disease before physiological manifestation set in.

8. The method as claimed in claim 5, wherein the human being is an individual with said mutation in heterozygous state.

9. A diagnostic kit useful for screening human beings for detecting a pre-disposition to schizophrenia by identifying a polymorphism A/G at nucleotide No. 825 from 5'end in exon 2 and its allelic variants in synaptagyrin 1 gene of chromosome 22q11-13, for detecting non-sense mutation of codon TGG coding for the amino acid tryptophan to a TAG, a non-sense codon, wherein said kit comprises:
(a) primers of SEQ ID No. 1-2;
(b) probes and/or primers of SEQ ID No. 3-7, and
(c) other additives selected from restriction enzymes, reverse transcriptases, polymerases, ligases, linkers, nucleoside triphosphates as substrate, suitable buffers, labels, and/or other accessories.

## Patentansprüche

1. Primer mit den SEQ ID Nos. 1 und 2.

2. Primer mit SEQ ID No. 3.

3. Primer und/oder Sonden mit den SEQ ID Nos. 4-7.

4. Verwendung der Primer nach Anspruch 1 zum Amplifizieren von Exon 2 des Synaptogyrin-1-Gens.

5. *In*-*vitro*-Verfahren zum Screenen von Menschen für eine Prädisposition für Schizophrenie durch Identifizieren eines Polymorphismus A/G am Nucleotid Nr. 825 von dem 5'-Ende in Exon 2 und seinen allelischen Varianten in dem Synaptogyrin-1-Gen des Chromosoms 22q11-13 zum Nachweisen von non-sense-Mutation des Codons TGG, codierend für die Aminosäure Tryptophan, zu einem TAG, einem non-sense-Codon, wobei das Verfahren umfasst:
(a) Isolieren von DNA aus Leukozyten des Blutes;
(b) Amplifizieren von isolierter DNA durch PCR unter Verwendung von Primern mit SEQ ID No. 1 und 2;
(c) Sequenzieren der amplifizierten DNA;
(d) Vergleichen der sequenzierten DNA mit der des normalen Synaptogyrin-1-Gens;
(e) Identifizierten der Mutation;
(f) Gestalten des Oligonucleotid primers und/oder der Sonde mit SEQ ID No. 3 der eingeschlossenen Sequenzauflistung, wobei sich sein 3'-Ende bis zu der vorletzten Position der Mutation erstreckt;
(g) Screenen für die non-sense-Mutation unter Verwendung des Primers und/oder der Sonde von Schritt (f): end
(h) Screenen der allelischen Variation für die non-sense-Mutation unter Verwendung von geeigneten Allel-spezifischen Oligonucleotid-Sonden und/oder -Primern, ausgewählt aus einer Gruppe, umfassend SEQ ID No. 4 bis 7 der eingeschlossenen Sequenzauflistung.

6. Verfahren nach Anspruch 5 zum Untersuchen von Vererbungsmustern in schizophrenen Familien.

7. Verfahren nach Anspruch 5, wobei das Verfahren frühen Nachweis erlaubt, um ein Management der Krankheit vor dem Einsetzen physiologischer Manifestation zu erlauben.

8. Verfahren nach Anspruch 5, wobei der Mensch ein Individuum mit der Mutation im heterozygoten Zustand ist.

9. Diagnostischer Kit, verwendbar zum Screenen von Menschen zum Nachweisen einer Prädisposition für Schizophrenie durch Identifizieren eines Polymorphismus A/G am Nucleotid Nr. 825 von dem 5'-Ende in Exon 2 und seinen allelischen Varianten in dem Synaptogyrin-1-Gen des Chromosoms 22q11-13 zum Nachweisen von non-sense-Mutation des Codons TGG. codierend für die Aminosäure Tryptophan, zu einem TAG, einem non-sense-Codon, wobei der Kit umfasst:
(a) Primer mit SEQ ID No. 1-2;
(b) Sonden und/oder Primer mit SEQ ID No. 3-7; und
(c) andere Zusatzstoffe, ausgewählt aus Restriktionsenzymen, reversen Transkriptasen, Polymerasen, Ligasen, Linkern, Nucleosidtriphosphaten als Substrat, geeigneten Puffern, Markierungen und/oderanderem Zubehör.

## Revendications

1. Les amorces des SEQ ID N° 1 et 2.

2. L'amorce de la SEQ ID N° 3.

3. Les amorces et/ou les sondes des SEQ ID N° 4-7.

4. Utilisation des amorces telles que revendiquées dans la revendication 1 pour amplifier l'exon 2 du gène de synaptogyrine 1.

5. Méthode *in vitro* de criblage pour une prédisposition à la schizophrénie chez des êtres humains en identifiant un polymorphisme A/G au niveau du nucléotide n° 825 de l'extrémité 5' dans l'exon 2 et ses variantes alléliques dans le gène de synaptogyrine 1 du chromosome 22q11-13, afin de détecter une mutation non-sens du codon TGG codant pour l'acide aminé tryptophane en un TAG, un codon non-sens. ladite méthode comprenant:
(a) isoler l'ADN de leucocytes du sang;
(b) amplifier par PCR l'ADN isolé, en utilisant les amorces des SEQ ID N° 1 et 2;
(c) sequencer l'ADN amplifié;
(d) comparer l'ADN séquencé à celui du gène normal de synaptogyrine 1;
(e) identifier ladite mutation;
(f) créer une amorce et/ou une sonde oligonucléotidique de la SEQ ID N° 3 de la liste des séquences ci-jointe, avec son extrémité 3' s'étendant jusqu'à l'avant-dernière position de ladite mutation;
(g) cribler pour ladite mutation non-sens en utilisant l'amorce et/ou la sonde de l'étape (f); et
(h) cribler ladite variation allélique pour ladite mutation non-sens en utilisant des sondes et/ou des amorces oligonucléotidiques appropriées spécifiques pour un allèle, sélectionnées parmi un groupe comprenant les SEQ ID N° 4 à 7 de la liste des séquences ci-jointe.

6. La méthode teille que revendiquée dans la revendication 5, pour étudier des modèles d'hérédité dans des familles schizophréniques.

7. La méthode telle que revendiquée dans la revendication. 5, laquelle méthode permet la détection précoce afin de permettre une prise en charge de la maladie avant qu'une manifestation physiologique ne s'établisse.

8. La méthode telle que revendiquée dans la revendication 5, où l'être humain est un individu présentant ladite mutation à l'état hétérozygote.

9. Kit de diagnostic utile pour faire un criblage pour détecter une prédisposition à la schizophrénie chez des êtres humains en identifiant un polymorphisme A/G au niveau du nucléotide n° 825 de l'extrémité 5' dans l'exon 2 et ses variantes alléliques dans le gène de synaptogyrine 1 du chromosome 22q11-13, afin de détecter une mutation non-sens du codon TGG codant pour l'acide aminé tryptophane en un TAG, un codon, non-sens, où ledit kit comprend:
(a) des amorces des SEQ ID N° 1-2;
(b) des sondes et/ou des amorces des SEQ ID N° 3-7; et
(c) d'autres additifs sélectionnés parmi des enzymes de restriction, des transcriptases inverses, des polymérases, des ligases, des lieurs, des nucléosides triphosphates en tant que substrats, des tampons appropriés, des marqueurs et/ou d'autres accessoires.


### SEQUENCE LISTING

<110> Council of Scientific and Industrial Research
<120> Novel primers for screening Schizophrenia and a method thereof
<130> US 799
<140>
   <141>
<160> 9
<170> PatentIn Ver. 2.1
<210> 1
   <211> 22
   <212> DNA
   <213> Artificial Sequence
<220>
   <223> Description of Artificial Sequence: primers for identifying and screening non-sense mutation in Schizophrenia
<400> 1
   ttgaagcagc tggcccgaat gt 22
<210> 2
   <211> 22
   <212> DNA
   <213> Artificial Sequence
<220>
   <223> Description of Artificial Sequence: primers for identifying and screening non-sense mutation in Schizophrenia
<400> 2
   tccccactct gagaccctga ac 22
<210> 3
   <211> 20
   <212> DNA
   <213> Artificial Sequence
<220>
   <223> Description of Artificial Sequence: primers for identifying and screening non-sense mutation in Schizophrenia
<400> 3
   acagaggtcg tgggtgagct 20
<210> 4
   <211> 27
   <212> DNA
   <213> Artificial Sequence
<220>
   <223> Description of Artificial Sequence: primers for identifying and screening non-sense mutation in Schizophrenia
<400> 4
   agctggacag aggtcgtggg tgagctg 27
<210> 5
   <211> 27
   <212> DNA
   <213> Artificial Sequence
<220>
   <223> Description of Artificial Sequence: Primers for identifying and screening non-sense mutation in schizophrenia
<400> 5
   agctggacag aggtcgtggg tgagcta 27
<210> 6
   <211> 27
   <212> DNA
   <213> Artificial Sequence
<220>
   <223> Description of Artificial Sequence:: primers for identifying and screening non-sense mutation in Schizophrenia
<400> 6
   gtcgtgggtg agctggagga gcaggcc 27
<210> 7
   <211> 27
   <212> DNA
   <213> Artificial Sequence
<220>
   <223> Description of Artificial Sequence:: primers for identifying and screening non-sense mutation in Schizophrenia
<400> 7
   gtcgtgggtg agctagagga gcaggcc 27
<210> 8
   <211> 20
   <212> DNA
   <213> Artificial Sequence
<220>
   <223> Description of Artificial Sequence: primers for identifying and screening non-sense mutation in schizophrenia
<400> 8
   ggcatgcctc cttggtctca 20
<210> 9
   <211> 19
   <212> DNA
   <213> Artificial Sequence
<220>
   <223> Description of Artificial Sequence: primers for identifying and screening non-sense mutation in Schizophrenia
<400> 9
   acatccgggc ctgctcctc 19